# EUROPEAN PATENT SPECIFICATION

(11) **EP 4 251 023 B1**
(45) Date of publication and mention of the grant of the patent: **31.12.2025**
(21) Application number: 21839691.9
(22) Date of filing: 25.11.2021
(51) Int. Cl.: A47L 15/00, A61L 2/22, A61L 2/24, B05B 3/00, B05B 13/00, A61L 2/16

(54) **MACHINE AND METHOD FOR THE TREATMENT OF OBJECTS, IN PARTICULAR WASHING AND SANITISING**
MASCHINE UND VERFAHREN ZUR BEHANDLUNG VON GEGENSTÄNDEN, INSBESONDERE ZUM WASCHEN UND DESINFIZIEREN
MACHINE ET PROCÉDÉ DE TRAITEMENT D'OBJETS, EN PARTICULIER DE LAVAGE ET DE DÉSINFECTION

(30) Priority: 26.11.2020 IT 202000028667
(43) Date of publication of application: 04.10.2023
(73) Proprietor: Steelco S.p.A., 31039 Riese Pio X (IT)
(72) Inventor: ZARDINI, Fabio, 31033 CASTELFRANCO VENETO (IT)
(74) Representative: Petraz, Davide Luigi
(86) International application number: PCT/IT2021/050381
(87) International publication number: WO 2022/113141

(56) References cited:
- WO-A1-2020/095335
- CN-A- 105 854 050
- US-A1- 2004 091 389
- US-A1- 2016 067 747
- US-A1- 2018 281 032
- US-A1- 2020 085 277

## Description

### FIELD OF APPLICATION

Embodiments disclosed herein relate to a machine for treating objects, in particular for washing and sanitizing, and to the relative treatment method. In particular, the present treatment machine is used in the field of healthcare, i.e., in a hospital, medical, pharmaceutical, laboratory context or the like for treating medical, hospital, surgical, laboratory, research, analysis instruments or the like or similar instruments, devices or components.

### BACKGROUND ART

Object treatment machines used to treat instruments used in the field of healthcare, in the laboratory, for analysis or for research, instruments used in the pharmaceutical sector, or instruments in the medical sector, surgical instruments or the like or similar instruments, hereinafter referred to as "objects", are known. Such items may be, for example, surgical or hospital devices or instruments, such as hospital pans, or other items, instruments or medical or health devices which require cleaning and/or disinfection, surgical instruments, laboratory or other instruments.

The term treatment can mean both pre-treatment operations of the objects, such as pre-cleaning, with hot or cold water and/or with detergents or other chemical products, as well as actual washing operations, and drying operations. The term "treatment" also comprises thermal-disinfection operations, for example sterilization, and decontamination by means of particularly aggressive and dangerous decontaminating substances, detergents and/or chemical agents, such as peracetic acid.

The washing and sanitizing of said objects must remove everything which is deposited on the object to be cleaned and must also possibly affect and in any case remove what may be present in the material thus deposited on the objects to be treated.

Different types of treatment fluids can be used for these operations, delivered by means of nozzles, for example, additive water can be used for washing and non-additive water for any rinsing operations of the washed objects and the treatment chamber.

An example of such machines is described in international application PCT/IT2019/050222 on behalf of the Applicant.

The volume of fluid used at each treatment cycle depends on several variables, including the size of the treatment chamber, the load of objects in the treatment chamber, the washing intensity, the number of delivery nozzles, washing time, etc.

The known machines generally deliver the processing fluid by means of nozzles which can be of two types: mobile, in particular rotating, or fixed.

The mobile nozzles are positioned on passive type rotating parts, which are configured to be rotated when crossed by a fluid to be delivered, generally supplied at a desired pressure typically related to the rotation speed which is desired to impart to the nozzle. The effect which is obtained is a rotary jet from the effective action of removing unwanted material from the objects to be washed and, therefore, such nozzles are generally used as main elements to obtain a complete cleaning of the objects.

The rotation of the mobile nozzles is, as mentioned, "passive", i.e., subject to the passage of the fluid inside the rotating member and its control can only be exercised by modifying the fluid feed.

The fixed nozzles, on the contrary, are configured to deliver the flow in a substantially non-modifiable direction.

One drawback of the known machines is the inefficient use of the treatment fluid which causes a waste of water during the course of the treatment cycle.

Such a negative aspect is due, in particular, to the poor optimization of the flows delivered by the nozzles both in terms of quantity delivered and in terms of delivery time with respect to the aforesaid variables, aspects connected, in part, to the technical features of the nozzles used.

In particular, the mobile nozzles, whose rotation is constrained to the fluid flow, can be inefficient from the point of view of fluid consumption. In fact, a high amount of fluid must be used to put them in rotation, generally in excess with respect to the real washing needs. Furthermore, since the rotational capacity of such mobile nozzles essentially depends on the fluid dispensed, their control is also difficult.

It is also known to deliver fluid from the nozzles simultaneously from all the available nozzles.

One drawback is that, especially in treatment chamber conditions only partially occupied by objects, some of the jets delivered by the nozzles may not affect any object, being substantially wasted.

Another drawback is that, by simultaneously feeding all the nozzles, the amount of liquid required for treatment increases while the pressure of the fluid in output from the nozzle decreases.

Therefore, there is a need to improve an object treatment machine and a relative method which, in the field of healthcare, i.e., in the hospital, medical, pharmaceutical, laboratory context or the like, can overcome at least one of the drawbacks of the art.

In particular, it is an object of the present invention to provide a machine and a method for treating objects which allow to optimize fluid consumption, in particular water consumption and total cycle time.

It is a further object of the present invention to provide a machine and a method for treating objects which allow the fluid consumption to be adapted to the needs of the treatment cycle.

It is another object of the present invention to provide an object treatment machine with controlled activation of the nozzles.

It is a further object is to devise a treatment method which allows to optimize the consumption of treatment fluid, in particular the consumption of water.

The Applicant has studied, tested and obtained the present invention to overcome the drawbacks of the prior art, and to obtain these and further objects and advantages.

### DISCLOSURE OF THE INVENTION

The present invention is expressed and characterised in the independent claims. The dependent claims show other features of the present invention or variants of the main solution idea.

In accordance with the aforesaid objects, a cleaning and/or sanitizing treatment machine is provided for objects in the field of healthcare, i.e., in the hospital, medical, pharmaceutical, research or analysis laboratories contexts or the like, in particular for washing, disinfecting and/or sterilizing. The treatment machine comprises a treatment chamber adapted to contain the objects to be treated and a feed circuit of at least one treatment fluid associated with a source of the aforesaid fluid, and a plurality of delivery nozzles arranged in the treatment chamber. Such items may be surgical or hospital devices or instruments, such as hospital pans, or items, instruments, or medical or healthcare devices which require cleaning and/or sanitization, surgical instruments, laboratory research or analysis instruments, or other.

According to a first aspect of the invention, the delivery nozzles comprise at least one main delivery nozzle motorized in rotation by means of connection to a respective motor member.

Advantageously, the need to consider the rotation speed as a function of the flow rate delivered is eliminated, allowing the two variables to be managed separately and independently. With this solution, it is therefore possible to obtain an optimal rotation speed for treatment with less use of treatment liquid than is achievable with the prior art.

According to a further aspect of the invention, such at least one main delivery nozzle is associated with a rotating member mechanically connected by a transmission member to said motor member and fluidically to said feed circuit.

According to a further aspect of the invention, said rotating member is configured to tilt with respect to said transmission member.

According to a further aspect of the invention, the machine comprises fixed nozzles and mobile nozzles and the feed circuit is configured to independently feed the at least one main delivery nozzle with respect to the fixed nozzles and the mobile nozzles.

In accordance with embodiments, the machine comprises a command and control unit connected to valves configured to control the delivery of the delivery nozzles.

With this solution it is possible to both select which nozzles or groups of nozzles must deliver and their flow rate.

According to a third aspect of the invention, the feed circuit comprises a pressure pump connected in inlet to the source and in outlet to the delivery nozzles, in which the pump is configured to generate a pressure between 3 and 7 bar, preferably between 3 and 6 bar, more preferably between 3 and 5 bar.

According to a further aspect of the invention, the machine comprises a microswitch connected to said motor member.

Such a microswitch can be connected to such a command and control unit.

According to a further aspect of the invention, the machine comprises at least one camera positioned inside said treatment chamber and configured to frame the object or objects to be treated.

Such a camera may be connected to such a command and control unit.

According to a further aspect of the invention, said feed circuit comprises a steam generator configured to perform a sanitizing step on the object or objects to be treated.

The main advantage of the treatment machine is the saving of water consumption per treatment cycle in view of reduced time for the end of the treatment cycle itself.

The reduction of water consumption also reduces the use of additives or other substances used in cleaning with the benefit of both the costs per treatment and the pollution generated by their use.

To understand the advantages of the invention, the Applicant reports, with reference to an intensive treatment cycle, a reduction in fluid consumption of about 75-80% in a third of the time with respect to the known machines.

A further object of the invention is a treatment method for cleaning and/or sanitizing objects in the field of healthcare, i.e., in a hospital, medical, pharmaceutical, laboratory context or the like, which includes, during a treatment cycle, delivering inside a treatment chamber at least one treatment fluid by a plurality of delivery nozzles comprising at least one main delivery nozzle motorized in rotation by connection to a respective motor member.

### ILLUSTRATION OF THE DRAWINGS

These and other aspects, features and advantages of the present invention will become clear from the following embodiment disclosure, given as a non-limiting example, with reference to the attached drawings in which:

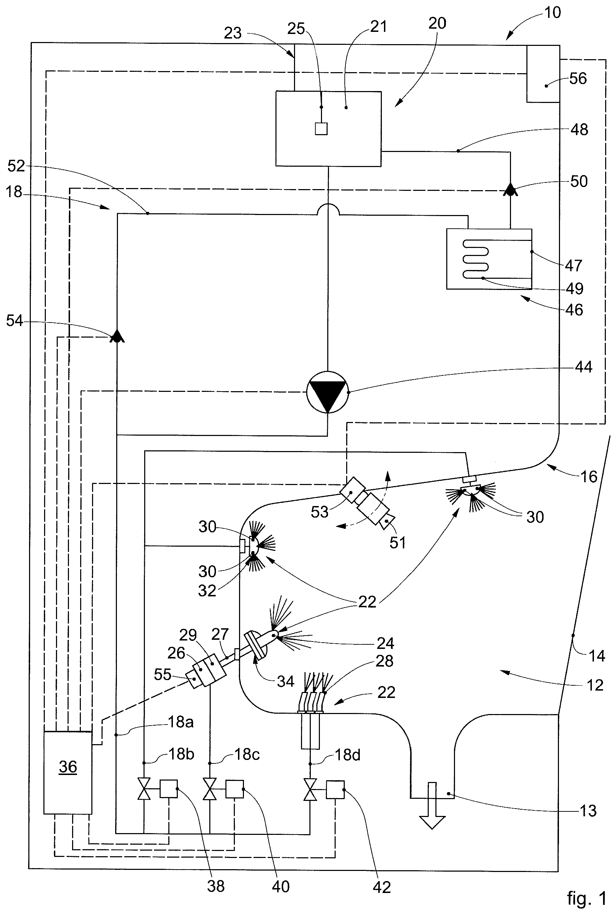

- fig. 1 is a schematic and very simplified illustration of a possible cleaning and sanitizing machine in accordance with the embodiments described herein.

To facilitate understanding, identical reference numbers have been used, where possible, to identify identical common elements in the figures. It is to be understood that elements and features of an embodiment can be conveniently combined or incorporated into other embodiments without further clarification.

### DESCRIPTION OF EMBODIMENTS

Reference will now be made in detail to the possible embodiments of the invention, one or more examples of which are shown in the attached figures by way of non-limiting example. The phraseology and terminology used herein is also for non-limiting exemplary purposes.

Referring to figure 1, the invention relates to a treatment machine 10 for cleaning and/or disinfecting objects, in particular washing, thermal-disinfecting and/or sterilizing. The objects in question are objects used in the field of healthcare, i.e., in a hospital, medical, pharmaceutical, laboratory context or similar fields. Such items may be surgical or hospital devices or instruments, such as hospital pans, or other items, instruments, or medical or healthcare devices which require cleaning and/or disinfection, surgical instruments, laboratory research or analysis instruments, or other.

In fig. 1 the dashed lines refer to electrical or and/or data connections, while the continuous lines, where reconcilable, refer to fluidic connections.

Here and in the present description, the term treatment can be understood as washing and/or activity by means of hot and/or cold liquids with or without the use of chemical agents, the treatment cycle itself being known and variable due to the type of hospital or laboratory product or product of other origin to be washed. The term treatment fluid will be used with reference to the liquid, or other form, which is used in the treatment cycles, which can be water, even in the form of steam, or possibly water added with chemical agents.

Different types of treatment fluids can be used in embodiments, for example additive water for washing and non-additive water for any rinsing operations can be used.

Furthermore, the term sanitizing can be understood as a washing, thermal-disinfecting and/or sterilizing treatment, typically by the use of water, controlled water, hot air and/or steam or in general cleaning fluids, liquids or aeriforms, for a predetermined or variable time depending on specific needs and cases.

The machine 10 comprises a treatment chamber 12 adapted to contain the objects to be treated and a door 14 configured to close an opening 16 of the treatment chamber 12.

The door 14 can be cantilevered and be associated with a closing device, not illustrated herein, such as that described in application IT102019000022080 on behalf of the Applicant.

The treatment of the objects occurs by a cleaning treatment cycle which can comprise a washing step for the removal of macroscopic dirt and/or a sanitizing step and, possibly, a reconditioning or cleaning step of the treatment chamber 12 to make it suitable for a subsequent cleaning treatment cycle.

Rinsing steps can be included between one step and the other, preferably with non-additive water.

The treatment chamber 12 comprises a drainage seat 13 placed at the bottom in the most appropriate position to allow an efficient outflow of the fluids during the treatment cycle to a tank or wastewater circuit (not shown).

The machine 10 comprises a feed circuit 18 fluidically associated with a source 20 of treatment fluid and a plurality of delivery nozzles 22, arranged inside the treatment chamber 12, for delivering the aforesaid treatment fluids and/or cleaning and/or sanitizing fluids.

The delivery nozzles 22, can be associated with the treatment chamber 12 by metallic coupling to reduce the risk of any fluid leakage, improving consumption and reducing any faults.

The delivery nozzles 22 can be of known type and comprise fixed nozzles 28 and mobile nozzles 30, the latter associated with a respective rotating member 32.

In embodiments, the source 20 can comprise a tank 21 of suitable capacity to contain a treatment fluid and connecting means 23 configured to supply water to the tank 21. The connecting means 23 can be configured to be in direct contact with the water mains or, alternatively, provide an inlet from which an operator can manually input water into the tank 21.

In variations, the connecting means 23 are provided with a valve configured to allow the entry of water when suitable sensors detect an amount of fluid in the tank 21 below a predetermined value.

The tank 21 can be connected to feed conduits, possibly associated with respective tanks of additive agents to be mixed to form the specific treatment fluids to the feed circuit 18.

The tank 21 can comprise at least one fill sensor 25 configured to detect the amount of fluid, for example a buoyancy sensor. The at least one fill sensor 25 can be connected to the command and control unit 36 to send data on the fill state of the tank 21 and possibly to control its filling.

According to a first aspect of the invention, the delivery nozzles 22 comprise at least one main delivery nozzle 24 motorized in rotation by connection to a respective motor member 26.

With respect to the mobile nozzles 30, whose rotation is dependent on the passage of the flow of treatment fluid, the at least one main delivery nozzle 24 is brought into rotation independently of the motor member 26.

One advantage is that the rotation speed of the at least one main delivery nozzle 24 can be selected independently of the flow rate of the jet, thus eliminating the proportionality constraint between the two variables in which the higher the flow rate, the higher the rotation speed. This solution translates into a more efficient use of the treatment fluid which means saving it and the possibility of obtaining an optimized rotation speed.

The at least one main delivery nozzle 24 can be associated with a rotating member 34 mechanically connected by a transmission member 27 to the motor member 26 and fluidically to the feed circuit 18.

The rotating member 34 can be configured to tilt with respect to the transmission member 27. It is thereby possible to position the rotating member 34 so as to direct the jet of the at least one main nozzle 24 towards a certain area of the treatment chamber 12.

In variations, the motor member 26 can comprise a gearmotor 29. In further variations, the motor member 26 can comprise a microswitch 55. The number of revolutions of the motor member 26 driving the main nozzle 24 preferably remains fixed, thus said main nozzle 24 rotates at a defined rotation speed. Therefore, the operation of the motor member 26 can be controlled by means of said microswitch 55, which can start or stop said motor member 26 at the defined speed, for example by means of the command and control unit 36.

In embodiments, the feed circuit 18 can be configured to independently feed the delivery nozzles 22. In particular, the feed circuit 18 can be configured to independently feed the at least one main nozzle 24 with respect to the fixed nozzles 28 and the mobile nozzles 30.

In a variant, the feed circuit 18 can be configured to also independently feed the fixed nozzles 28 and the mobile nozzles 30.

In the example case illustrated in figure 1, the feed circuit 18 comprises a main branch 18a in output from the source 20 which is divided into three secondary branches 18b, 18c and 18d each associated respectively with a group of mobile nozzles 30, the main delivery nozzle 24 or a group of main nozzles 24, and a group of fixed nozzles 28. Such a solution efficiently allows the fluid to be distributed, possibly selectively, to said group of mobile nozzles 30, to the main delivery nozzle 24 or said group of main nozzles 24, and to said group of fixed nozzles 28.

At least one secondary branch 18b, 18c or 18d, preferably each thereof, can comprise a respective valve 38, 40, 42 configured to control the delivery of the delivery nozzles 22.

In possible variations, the valves 38, 40 and 42 are configured to be positioned in an open position, allowing fluid to pass through or in a closed position, preventing fluid from passing through.

In other possible variations, the valves 38, 40, and 42 can be proportional valves configured to adopt more than one position between the open position and the closed position to adjust the flow rate.

In variants, the valves 38, 40, 42 are solenoid valves.

According to a second aspect of the invention, the machine 10 comprises a command and control unit 36 connected to the valves 38, 40 and 42.

The command and control unit 36 is configured to control the opening of the valves 38, 40 and 42, in a manner coordinated with the type of cleaning and/or sanitizing treatment. Since, advantageously, each valve controls the flow to respective groups of delivery nozzles 22, it is possible to select which group of delivery nozzles 22 are to be actuated at a given time of the treatment cycle and possibly control the flow rate of each flow.

Such a solution positively allows to adapt the consumption of treatment fluid consistently to the needs or conditions of the treatment cycle, according to variables such as the load of objects to be washed in the treatment chamber and possibly their position, the type and extent of the dirt (liquid or encrusted), the step of the cycle, etc.

Such a command and control unit 36 is connected to the motor member 26, so as to properly control the main nozzle 24, by connecting to the microswitch 55 connected to said motor member 26.

In embodiments, the at least one main nozzle 24 is used at least in the washing step where dirt needs to be removed from objects.

In the rinsing step, where high pressures are not required, the at least one main nozzle 24 can be isolated from the feed circuit 18 by closing the valve 40 and the motor 26 is kept off. Jointly, the valves 38 and/or 42 are at least partially open to allow fluid to flow into the branches 18b and 18d, respectively.

According to a third aspect of the invention, the feed circuit 18 comprises a pressure pump 44, connected in inlet to the source 20 and in outlet to the delivery nozzles 22 configured to generate a pressure between 3 and 7 bar, preferably between 3 and 6 bar, more preferably between 3 and 5 bar.

In the machine illustrated in figure 1, the pump 44 is connected in inlet to the tank 21.

The pump 44 is connected to the command and control unit 36 which controls the power delivery thereof in coordination with the needs of the treatment cycle.

By intervening on the power fed by the pump 44 it is possible to change the flow rate towards the nozzles 22. Therefore, in accordance with possible variations, it is possible to close the fluid delivery to certain nozzles 22 by closing respective valves and changing the flow rate to the nozzles 22 of respective open valves by adjusting the power of the pump 44.

In embodiments, the feed circuit 18 comprises a steam generator 46 configured to perform a sanitizing step of the object or objects to be treated.

The steam generator 46 can be a boiler 47, preferably electric, provided with a heating device 49, for example a coil, advantageously placed in a position which leads it to be in contact with the fluid when present, and configured to be brought to a suitable temperature to generate steam when directly or indirectly in contact with the aforementioned fluid.

In the case illustrated in figure 1, the steam generator 46 is fluidically connected to the tank 21 by a conduit 48 for receiving water in the liquid state. The conduit 48 can be provided with a valve 50, optionally connected to the command and control unit 36, configured to control the flow of water from the source 20.

The steam generator 46 is connected to the feed circuit 18 by means of a conduit 52. The conduit 52 can be provided with a valve 54, optionally connected to the command and control unit 36, configured to allow the inflow of steam to the feed circuit 18.

The valves 50 and 54 are advantageously non-return valves.

The conduit 52 connects to the feed circuit 18 bypassing the pump 44.

The command and control unit 36 can comprise a memory in which predetermined programs or treatment settings are saved which allow the treatment cycle to be automatically managed according to specific needs or conditions, for example, the load of objects to be washed in the treatment chamber, the type and extent of the dirt, the function of each step of the cycle, etc.

The command and control unit 36 allows to partially, i.e., alternately select or deselect, the actuation of the valves 38, 40 and 42 and, therefore, control the jet of the delivery nozzles 22.

Therefore, the jet of each group of delivery nozzles 22 can be interrupted, decreased or increased during the treatment cycle according to appropriate control sequences.

Jointly, the command and control unit 36 can control the operating parameters of the pump 44, for example the power delivered to regulate the fluid pressure within the feed circuit 18 and thus provide a further mode for changing the flow rate.

In the washing step it is envisaged to operate the pump 44 which introduces treatment fluid into the conduit 18a at a predetermined pressure, the valves 50 and 54 are closed to exclude the steam generator 46.

In the sanitizing step, it is envisaged to introduce steam into the conduit 18a by opening the valve 54. The valve 50 can be opened to provide water to the boiler 47 which generates the necessary steam.

A user interface 56, which can comprise a touch screen, allows an operator to select the predetermined programs or, alternatively, manually and punctually select operating parameters, if any, for each step of the treatment cycle before launching the treatment cycle.

The present machine 10 can also comprise at least one camera 51 positioned inside the treatment chamber 12, so as to frame the object or objects to be treated.

Said camera 51 can be connected to a motor member 53 which allows it to rotate around one or more rotation axes, in order to correctly frame the areas inside said treatment chamber 12.

Said camera 51 can be connected to the command and control unit 36 and thus transmit images related to the object or objects to be treated thereto. Thereby, the command and control unit 36 will be able to selectively operate the nozzles 22 depending on the features of the object or objects to be washed, for example shape, size, number of objects if more than one, or other. Furthermore, by means of the camera 51, it is possible to check the progress of the treatment process or even if the process occurs regularly inside the treatment chamber 12, i.e., if any problem occurs during the treatment. Said camera 51 can be connected to the user interface 56, which can be provided with a display or the like.

Embodiments also relate to a treatment method for cleaning and/or sanitizing objects, in particular washing, thermal-disinfecting and/or sterilizing objects.

According to a first aspect, during a treatment cycle, the method includes delivering inside the treatment chamber 12 a treatment fluid by a plurality of delivery nozzles 22 comprising at least one main delivery nozzle 24 motorized in rotation by connection to a respective motor member 26.

According to a second aspect, the delivery nozzles 22 can comprise fixed nozzles 28 and mobile nozzles 30, and the method includes independently feeding the at least one main delivery nozzle 24 with respect to the fixed nozzles 28 and mobile nozzles 30.

In embodiments, the method includes, by using the valves 38, 40, 42 connected to the command and control unit 36, partializing the flow of the delivery nozzles 22 consistent with the needs of the treatment cycle.

In embodiments, when the treatment cycle includes using the at least one main delivery nozzle 24, the valve 40 is at least partially opened to allow fluid delivery and the motor 26 can be driven to provide flow rotation. The pump 44 is in operation and delivers a certain power suitable to provide a desired pressure to the treatment fluid to be delivered.

One or both of the valves 38 and 42 can be closed or at least partially opened as needed.

The valves 38, 40, 42 can be controlled to obtain a delivery of the respective delivery nozzles 22 which can follow a certain pattern, for example sequential or alternating, linked for example to their position in the treatment chamber 12 and/or their type (fixed or rotating) and/or the type of cleaning and/or sanitizing treatment.

Depending on the opening of the valves 38, 40, 42, the pump 44 can change its operating power to maintain the flow pressure delivered by the nozzles 22 constant.

It is clear that modifications and/or additions of parts may be made to the treatment method described so far, without departing from the scope of the present invention as defined by the claims.

In the following claims, the references in parentheses have the sole purpose of facilitating reading and must not be considered as limiting factors as regards the scope of protection underlying the specific claims.

## Claims

1. Machine (10) for the cleaning and/or sanitizing treatment of objects in the field of healthcare, i.e., in a hospital, medical, pharmaceutical, laboratory context or the like, the machine comprising a treatment chamber (12) adapted to contain the objects to be treated and a circuit (18) for feeding at least one treatment fluid associated with a source (20) of said fluid and a plurality of delivery nozzles (22) arranged in said treatment chamber (12),
**characterized in that** said delivery nozzles (22) comprise at least one main delivery nozzle (24) motorized in rotation by connection to a respective motor member (26), wherein said delivery nozzles (22) comprise fixed nozzles (28) and mobile nozzles (30), said feed circuit (18) being configured to feed said at least one main delivery nozzle (24) in an independent manner with respect to said fixed nozzles (28) and said mobile nozzles (30), and wherein with respect to the mobile nozzles (30), whose rotation is dependent on the passage of the flow of treatment fluid, the at least one main delivery nozzle (24) is brought into rotation independently by the motor member (26).

2. Machine (10) according to claim 1, **characterized in that** said at least one main delivery nozzle (24) is associated with a rotating member (34) mechanically connected by a transmission member (27) to said motor member (26) and fluidically connected to said feed circuit (18).

3. Machine (10) according to claim 2, **characterized in that** said rotating member (34) is configured to incline with respect to said transmission member (27).

4. Machine (10) according to one, or the other, of the preceding claims, **characterized in that** said machine (10) comprises a command and control unit (36) connected to valves (38, 40, 42) configured to control the delivery of said delivery nozzles (22).

5. Machine (10) according to one, or the other, of the preceding claims, **characterized in that** said feed circuit (18) comprises a pressure pump (44) connected in inlet to said source (20) and in outlet to said delivery nozzles (22), wherein said pump (44) is configured to generate a pressure between 3 and 7 bar, preferably between 3 and 6 bar, more preferably between 3 and 5 bar.

6. Machine (10) according to one, or the other, of the preceding claims, **characterized in that** it comprises a microswitch (55) connected to said motor member (26).

7. Machine (10) according to claims 4 and 6, **characterized in that** said microswitch (55) is connected to said command and control unit (36).

8. Machine (10) according to one, or the other, of the preceding claims, **characterized in that** it comprises at least one camera (51) positioned inside said treatment chamber (12) and configured to frame the object or objects to be treated.

9. Machine (10) according to claims 4 and 8, **characterized in that** said camera (51) is connected to the command and control unit (36).

10. Machine (10) according to one, or the other, of the preceding claims, **characterized in that** said feed circuit (18) comprises a steam generator (46) configured to perform a sanitizing step on the object or objects to be treated.

11. Treatment method for cleaning and/or sanitizing objects in the field of healthcare, i.e., in a hospital, medical, pharmaceutical context or the like, wherein during a treatment cycle, delivering, within a treatment chamber (12), at least one treatment fluid by a plurality of delivery nozzles (22) and being **characterized in that** it comprises at least one main delivery nozzle (24) motorized in rotation by a connection to a respective motor member (26), wherein said delivery nozzles (22) comprise fixed nozzles (28) and mobile nozzles (30), said feed circuit (18) being configured to feed said at least one main delivery nozzle (24) in an independent manner with respect to said fixed nozzles (28) and said mobile nozzles (30), and wherein with respect to the mobile nozzles (30), whose rotation is dependent on the passage of the flow of treatment fluid, the at least one main delivery nozzle (24) is brought into rotation independently by the motor member (26).

12. Method according to claim 11, **characterized in that** it includes, by means of the use of valves (38, 40, 42) connected to a command and control unit (36), partializing the flow of said delivery nozzles (22) consistent with the needs of the treatment cycle.

13. Method according to one, or the other, of claims 11 or 12, **characterized in that** it includes delivering said treatment fluid at a pressure between 3 and 7 bar, preferably between 3 and 6 bar, more preferably between 3 and 5 bar.

## Patentansprüche

1. Maschine (10) für die Reinigungs- und/oder Desinfektionsbehandlung von Objekten aus dem Gebiet des Gesundheitswesens, d.h., in einem Krankenhaus-, Medizin-, Pharma-, Laborkontext oder dergleichen, wobei die Maschine folgendes umfasst:
eine Behandlungskammer (12), die dazu angepasst ist, das zu behandelnde Objekt aufzunehmen, und einen Kreislauf (18) für das Zuführen mindestens einer Behandlungsflüssigkeit, der zu einer Quelle (20) der Flüssigkeit und zu einer Vielzahl von Lieferdüsen (22) zugeordnet ist, die in der Behandlungskammer (12) angeordnet sind, **dadurch gekennzeichnet, dass**
die Lieferdüsen (22) mindestens eine Hauptlieferdüse (24) umfassen, die durch eine Verbindung mit einem jeweiligen Motorelement (26) rotatorisch motorisiert ist, wobei die Lieferdüsen (22) feste Düsen (28) und bewegliche Düsen (30) umfassen, wobei der Zuführkreislauf (18) so konfiguriert ist, dass er die mindestens eine Hauptlieferdüse (24) in Bezug zu den festen Düsen (28) und den beweglichen Düsen (30) in einer unabhängigen Weise zuführt, und wobei in Bezug zu den beweglichen Düsen (30), deren Rotation von dem Durchgang des Durchflusses der Behandlungsflüssigkeit abhängig ist, die mindestens eine Hauptlieferdüse unabhängig (24) durch das Motorelement (26) in Rotation gebracht ist.

2. Maschine (10) nach Anspruch 1, **dadurch gekennzeichnet, dass** die mindestens eine Hauptlieferdüse (24) zu einem Rotationselement (34) zugeordnet ist, das durch ein Getriebeelement (27) mit dem Motorelement (26) mechanisch verbunden ist und mit dem Zuführkreislauf (18) hydraulisch verbunden ist.

3. Maschine (10) nach Anspruch 2, **dadurch gekennzeichnet, dass** das Rotationselement (34) so konfiguriert ist, dass es in Bezug auf das Getriebeelement (27) geneigt ist.

4. Maschine (10) nach einem der vorhergehenden Ansprüche, **dadurch gekennzeichnet, dass** die Maschine (10) eine Befehls- und Steuereinheit (36) umfasst, die mit Ventilen (38, 40, 42) verbunden ist, die so konfiguriert sind, dass sie die Lieferung der Lieferdüsen (22) steuern.

5. Maschine (10) nach einem der vorhergehenden Ansprüche, **dadurch gekennzeichnet, dass** der Zuführkreislauf (18) eine Druckpumpe (44) umfasst, die am Einlass mit der Quelle (20) und am Auslass mit der Lieferdüse (22) verbunden ist, wobei die Pumpe (44) so konfiguriert ist, dass ein Druck zwischen 3 und 7 Bar, vorzugsweise zwischen 3 und 6 Bar, besonders vorzugsweise zwischen 3 und 5 Bar erzeugt ist.

6. Maschine (10) nach einem der vorhergehenden Ansprüche, **dadurch gekennzeichnet, dass** sie einen Mikroschalter (55) umfasst, der mit dem Motorelement (26) verbunden ist.

7. Maschine (10) nach den Ansprüche 4 und 6, **dadurch gekennzeichnet, dass** der Mikroschalter (55) mit der Befehls- und Steuereinheit (36) verbunden ist.

8. Maschine (10) nach einem der vorhergehenden Ansprüche, **dadurch gekennzeichnet, dass** sie mindestens eine Kamera (51) umfasst, die innerhalb der Behandlungskammer (12) positioniert ist und so konfiguriert ist, dass sie das Objekt oder die Objekte, die zu behandeln sind, einrahmt.

9. Maschine (10) nach den Ansprüche 4 und 8, **dadurch gekennzeichnet, dass** die Kamera (51) mit der Befehls- und Steuereinheit (36) verbunden ist.

10. Maschine (10) nach einem der vorhergehenden Ansprüche, **dadurch gekennzeichnet, dass** der Zuführkreislauf (18) einen Dampfgenerator (46) umfasst, der so konfiguriert ist, dass er einen Desinfektionsschritt an dem Objekt oder den Objekten, die zu behandeln sind, ausführt.

11. Verfahren zur Reinigungs- und/oder Desinfektionsbehandlung von Objekten aus dem Gebiet des Gesundheitswesens, d.h., in einem Krankenhaus-, Medizin-, Pharma-, Laborkontext oder dergleichen, wobei während eines Behandlungszyklus, in einer Behandlungskammer (12), mindestens eine Behandlungsflüssigkeit durch eine Vielzahl von Lieferdüsen (22) geliefert wird und **dadurch gekennzeichnet, dass**
mindestens eine Hauptlieferdüse (24) umfasst, die durch eine Verbindung mit einem jeweiligen Motorelement (26) rotatorisch motorisiert ist, wobei die Lieferdüsen (22) feste Düsen (28) und bewegliche Düsen (30) umfassen, wobei der Zuführkreislauf (18) so konfiguriert ist, dass er die mindestens eine Hauptlieferdüse (24) in Bezug zu den festen Düsen (28) und den beweglichen Düsen (30) in einer unabhängigen Weise zuführt, und wobei in Bezug zu den beweglichen Düsen (30), deren Rotation von dem Durchgang des Durchflusses der Behandlungsflüssigkeit abhängig ist, die mindestens eine Hauptlieferdüse (24) unabhängig durch das Motorelement (26) in Rotation gebracht wird.

12. Verfahren nach Anspruch 11, **dadurch gekennzeichnet, dass** es, mittels der Verwendung von Ventilen (38, 40, 42), die mit einer Befehls- und Steuereinheit (36) verbunden sind, das Aufteilen des Durchflusses der Lieferdüsen (22) konsistent mit dem Bedarf des Behandlungszyklus beinhaltet.

13. Verfahren nach einem der Ansprüche 11 oder 12, **dadurch gekennzeichnet, dass** es das Liefern der Behandlungsflüssigkeit mit einem Druck zwischen 3 bis 7 Bar, vorzugsweise mit 3 bis 6 Bar, besonders vorzugsweise zwischen 3 und 5 Bar, aufweist.

## Revendications

1. Machine (10) pour le nettoyage et/ou le traitement de désinfection d'objets dans le domaine de la santé, c'est-à-dire dans un contexte hospitalier, médical, pharmaceutique, de laboratoire ou similaire, **caractérisée en ce qu'**elle comprend une chambre de traitement (12) adaptée à contenir les objets à traiter et un circuit d'alimentation (18) pour alimenter au moins un fluide de traitement associé à une source (20) dudit fluide et une pluralité de buses de distribution (22) disposées dans ladite chambre de traitement (12), où lesdites buses de distribution (22) comprennent au moins une buse principale de distribution (24) motorisée en rotation par connexion à un membre moteur (26) respectif, où lesdites buses de distribution (22) comprennent des buses fixes (28) et des buses mobiles (30), ledit circuit d'alimentation (18) étant configuré pour alimenter ladite buse principale de distribution (24) de manière indépendante par rapport auxdites buses fixes (28) et auxdites buses mobiles (30), et où par rapport aux buses mobiles (30), dont la rotation dépend du passage du flux de fluide de traitement, la buse principale de distribution (24) est mise en rotation indépendamment par le membre moteur (26).

2. Machine (10) selon la revendication 1, **caractérisée en ce que** ladite buse principale de distribution (24) est associée à un élément rotatif (34) connecté mécaniquement par un élément de transmission (27) à ledit membre moteur (26) et connecté via les fluides audit circuit d'alimentation (18).

3. Machine (10) selon la revendication 2, **caractérisée en ce que** ledit élément rotatif (34) est configuré pour s'incliner par rapport audit élément de transmission (27).

4. Machine (10) selon l'une ou l'autre des revendications précédentes, **caractérisée en ce que** ladite machine (10) comprend une unité de commande et de contrôle (36) connectée à des vannes (38, 40, 42) configurées pour contrôler la distribution desdites buses de distribution (22).

5. Machine (10) selon l'une ou l'autre des revendications précédentes, **caractérisée en ce que** ledit circuit d'alimentation (18) comprend une pompe à pression (44) connectée en entrée à ladite source (20) et en sortie auxdites buses de distribution (22), où ladite pompe (44) est configurée pour générer une pression entre 3 et 7 bars, de préférence entre 3 et 6 bars, plus préférablement entre 3 et 5 bars.

6. Machine (10) selon l'une ou l'autre des revendications précédentes, **caractérisée en ce qu'**elle comprend un microswitch (55) connecté audit membre moteur (26).

7. Machine (10) selon les revendications 4 et 6, **caractérisée en ce que** ledit microswitch (55) est connecté à ladite unité de commande et de contrôle (36).

8. Machine (10) selon l'une ou l'autre des revendications précédentes, **caractérisée en ce qu'**elle comprend au moins une caméra (51) positionnée à l'intérieur de ladite chambre de traitement (12) et configurée pour cadrer l'objet ou les objets à traiter.

9. Machine (10) selon les revendications 4 et 8, **caractérisée en ce que** ladite caméra (51) est connectée à l'unité de commande et de contrôle (36).

10. Machine (10) selon l'une ou l'autre des revendications précédentes, **caractérisée en ce que** ledit circuit d'alimentation (18) comprend un générateur de vapeur (46) configuré pour effectuer une étape de désinfection sur l'objet ou les objets à traiter.

11. Procédé de traitement pour le nettoyage et/ou la désinfection d'objets dans le domaine de la santé, c'est-à-dire dans un contexte hospitalier, médical, pharmaceutique ou similaire, **caractérisé en ce qu'**il comprend, au cours d'un cycle de traitement, la distribution, à l'intérieur d'une chambre de traitement (12), d'au moins un fluide de traitement par une pluralité de buses de distribution (22) comprenant au moins une buse principale de distribution (24) motorisée en rotation par connexion à un membre moteur (26) respectif, où lesdites buses de distribution (22) comprennent des buses fixes (28) et des buses mobiles (30), ledit circuit d'alimentation (18) étant configuré pour alimenter ladite buse principale de distribution (24) de manière indépendante par rapport auxdites buses fixes (28) et auxdites buses mobiles (30), et où par rapport aux buses mobiles (30), dont la rotation dépend du passage du flux de fluide de traitement, la buse principale de distribution (24) est mise en rotation indépendamment par le membre moteur (26).

12. Procédé selon la revendication 11, **caractérisé en ce qu'**il comprend, par l'utilisation de vannes (38, 40, 42) connectées à une unité de commande et de contrôle (36), la régulation du flux desdites buses de distribution (22) conformément aux besoins du cycle de traitement.

13. Procédé selon l'une ou l'autre des revendications 11 ou 12, **caractérisé en ce qu'**il comprend la distribution dudit fluide de traitement à une pression entre 3 et 7 bars, de préférence entre 3 et 6 bars, plus préférablement entre 3 et 5 bars.
